Europäisches Patentamt

European Patent Office

Office européen des brevets

(11) Publication number: 0 400 931
A1

# EUROPEAN PATENT APPLICATION

(21) Application number: 90305756.0

(51) Int. Cl.5: C12N 15/74

(22) Date of filing: 25.05.90

(30) Priority: 29.05.89 JP 132686/89

(43) Date of publication of application:
05.12.90 Bulletin 90/49

(84) Designated Contracting States:
DE FR GB IT

(71) Applicant: NIPPON OIL CO., LTD.
3-12, Nishi-Shimbashi 1-chome Minato-ku
Tokyo(JP)

(72) Inventor: Yata, Tetsuhisa
17-25, Shinjyou-Nakamachi, Nakahara-ku
Kawasaki-shi, Kanagawa(JP)
Inventor: Maruhashi, Kenji
155-72, Honmokuohsato-cho, Naka-ku
Yokohama-shi, Kanagawa(JP)
Inventor: Kiyota, Takashi
94-9, Ichisawa-cho, Asahi-ku
Yokohama-shi, Kanagawa(JP)

(74) Representative: Davies, Jonathan Mark et al
Reddie & Grose 16 Theobalds Road
London WC1X 8PL(GB)

(54) Plasmid pTY1.

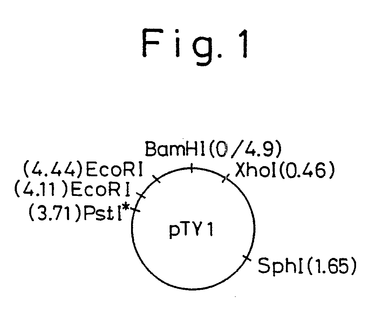

(57) Plasmid pTY1 having a molecular weight of about 4.9 mega Dalton and defined by a restriction enzyme cleavage map shown in Fig. 1, and obtainable from cells of Propionibacterium pentosaceum ATCC 4875 (IFO 12425) (FERM BP-2931).

Fig. 1

BamHI(0/4.9)

(4.44)EcoRI

(4.11)EcoRI

(3.71)PstI*

Xhol(0.46)

pTY1

SphI(1.65)

EP 0 400 931 A1

## PLASMID pTY1

The present invention relates to a novel plasmid pTY1 capable of replicating in bacteria belonging to Propionibacterium.

Recently, many DNA recombination experiments are underway using Escherichia coli, and various vectors able to be used in E. coli cells have been developed. In addition to this E. coli, Bacillus usable for the production of amylase enzymes and yeast usable in the alcohol drink industry are industrially important, and therefore, many attempts have been made to establish DNA recombination techniques for these industrial microorganisms. Further, since vectors are essential to the establishment of DNA recombination techniques, various plasmids and viruses have been screened to construct vectors.

On the other hand, since propionic acid bacteria have abilities to produce various kinds of biologically active substances, they have long been considered very important. Nevertheless, the means of breeding propionic acid bacteria are very limited, time-consuming, and laborious, and therefore, any improvement of the methods of breeding propionic acid bacteria is difficult. Accordingly, although there is an urgent need for the development of host-vector systems usable for gene recombination techniques in propionic acid bacteria, plasmids self-replicative in propionic acid bacteria are not as yet known, and therefore, the breeding of propionic acid bacteria remains difficult.

Therefore, the present invention provides a novel plasmid, pTY1, capable of replicating in cells of bacteria belonging to the genus Propionibacterium. The plasmid has a size of about 4.9 mega Dalton, is not cleaved by restriction enzymes Hind III, Bgl II, Pst I and Xba I, and is characterized by the restriction enzyme map shown in Fig. 1.

Figure 1 shows a restriction enzyme cleavage map of the plasmid pTY1, wherein the numbers in parentheses show the distance (clockwise) from the BamH I site.

The present plasmid pTY1 is derived from cells of bacteria belonging to Propionibacterium; for example, Propionibacterium pentosaceum ATCC 4875 (IFO 12425) can be used as a source of this plasmid. Prop. pentosaceum ATCC 4875 (IFO 12425) is stored in American Type Culture Collection (ATCC) and listed in the American Type Culture Collection Catalogue of Strains I, Fourteenth Edition, 1980, and is available to the public without limitation. The strain identification given by the depositor was Prop. pentosaceum TY-1. Moreover, this is stored in the Institute for Fermentation, Osaka (IFO), 17-85, Juso-honmachi 2-chome, Yodogawa-ku, Osaka 532, Japan, and listed in the catalogue, Institute for Fermentation, Osaka, List of Cultures, 1984, and is available to the public without limitation. Note, the strain Propionibacterium pentosaceum ATCC 4875 (IFO 1242S) was deposited with Fermentation Research Institute Agency of Industrial and Technology, 1-1, Higashi 1-chome Tsukuba-shi Ibaraki-ken 305, Japan, as FERM BP 2931, on May 19, 1990, under the Budapest treaty.

For culturing a bacterium of the genus Propionibacterium, any medium in which a desired bacterium grows while maintaining the plasmid pTY1 in a cell can be used. The composition of an example of such a medium is shown in Table 1.

Table 1

| Ingredients | Amount |
|---|---|
| Yeast extract | 8.5 g/l |
| Peptone | 8.5 g/l |
| Glucose | 11.0 g/l |
| KH$_2$PO$_4$ | 2.0 g/l |
| Tomato juice powder | 3.7 g/l |
| Tween 80 | 1.0 g/l |
| pH value | 6.8 |

Preferably, the culture temperature is 15°C to 42°C, more preferably about 24°C to 35°C, and the starting pH of the culture medium is about 6.0 to 8.0, more preferably about 6.5 to 7.5. Preferably, the culturing is carried out for 1 to 4 days, more preferably about 1 to 2 days.

To isolate a desired plasmid, cultured bacterial cells are collected by a conventional procedure such as centrifugation or filtration, and the collected cells are lyzed. Note, the cells of Propionibacterium can be lyzed by a lytic enzyme such as lysozyme, and if necessary, in addition to the lytic enzyme, another enzyme such as protease, or surfactant such as Sarcosyl or sodium lauryl sulfate can be added to the lytic mixture. Further, a treatment with penicilline G or freezing-thawing process may be used to accelerate the lysis of cells.

Thereafter, the plasmid can be isolated from the lysate prepared as above, by a conventional method such as a precipitation of DNA with ethanol, a cesium chloride density-gradient centrifugation method using ethidium bromide, a sucrose density-gradient centrifugation method, affinity chromatography, hydroxyapatite chromatography, gel electrophoresis, or dialysis using, for example, cellophane, or a combination of these processes.

To prepare a restriction enzyme cleavage map of the present plasmid pTY1, the plasmid is cleaved with various restriction enzymes. To this end, the plasmid is cleaved with an excess amount of a single restriction enzyme or a combination of restriction enzymes, and the cleavage product is subject to 0.6% agarase gel electrophoresis to determine the number of resulting fragments and the size (molecular weight) of each fragment. The molecular weight of the fragments is determined by comparing the mobility of the fragment with those of molecular weight markers prepared by cleaving λ phage DNA with Hind III (J. Mol. Biol. 98, 551-564, 1975). Where a single restriction enzyme provides more than one fraction, the values of the molecular weights of the fragments are summed, to determine a molecular weight of the whole plasmid.

The above-mentioned experiment showed that the plasmid pTY1 has the following cleavage sites for various restriction enzymes.

| Restriction enzyme | Number of cleavage sites |
|---|---|
| BamH I | 1 |
| EcoR I | 2 |
| Sph I | 1 |
| Xho I | 1 |
| Pst I(*) | 1 |
| Hind III | 0 |
| Bgl II | 0 |
| Pst I | 0 |
| Xba I | 0 |

(*) Note, although the plasmid pTY1 per se is not cleaved with Pst I, after it is inserted to an E. coli vector and the recombinant vector is replicated in E. coli, the r plicated vector contains a Pst I cleavage site in the pTY1 insert region.

A restriction enzyme cleavage map is determined from the above results, as shown in Fig. 1.

Note, because the present plasmid has cleavage sites for various restriction enzymes, it can be used to construct various useful vectors, and further, a desired gene can be inserted into the present plasmid or derivatives thereof to construct a recombinant vector, which is then used to incorporate the desired gene into an appropriate bacterial host. In particular, the present plasmid and derivatives thereof can be used as transformation vectors for industrially important microorganisms such as propionic acid bacteria, for example, bacteria belonging to Propionibacterium. For example, a gene from a propionic acid bacterium can be cloned in the present plasmid or a derivative thereof, and the cloned gene can be introduced into other microorganisms such as E. coli or B. subtilis, directly or indirectly through other vectors, and thus a useful product such as an antibiotic, a physiologically active substance or vitamin, originally produced by propionic acid bacteria, is produced in another microorganism such as E. coli, more efficiently than in the original propionic acid bacteria.

Also, the present plasmid and derivative thereof can be used to clone genes from higher animals or plants, such as a gene coding for somatostatin, a gene coding for insulin, or genes relating to nitrogen fixation.

The above-mentioned construction of various vectors can be carried out according to procedures well-known per se, for example, by the procedures described in Scientific American, Vol 233, No. 1, pp 24 to 33, 1975.

The present plasmid and derivatives thereof can be introduced into cells of an appropriate host by a calcium chloride method wherein calcium is used to increase the permeability of the cell wall, as reported for E. coli K-12 (Mandel, M. and Higa, A., J. Mol. Biol., 53, 159, 1970); a method using a competent state occurring at a stage of a cell-growing cycle as reported for B. subtilis (Duncan, C.H., Wilson, G.A. and Young, F.E., Gene 1, 153 (1977); and a protoplast or spheraplast method reported for B. subtilis, actinomyces and yeast (Chang, S. and Cohen, S.N., Molec. Gen. Genet. 168, 111, 1979; Bibb, M.J., Ward, J.M., and Hopwood, D.A., Nature, 274, 398, 1978; and Hinnen, A., Hicks, J.B. and Fink, G.R., Proc. Natl. Acad. Sci., USA, 75, 1929, 1978).

Host cells transformed with a recombinant vector derived from the present plasmid are cultured by a conventional method to produce a desired substance in a medium or in the cells, and the desired substance can be recovered and purified from the medium or cells by a conventional purification process.

The present plasmid may be used as a vector not only in host-vector systems wherein the host is propionic acid bacteria, but also in host-vector systems wherein the host is other gram positive bacterium such as Bacillus, Corynebacterium, or Brevibacterium.

## Example

The present invention will now by further illustrated by, but is by no means limited to, the following example.

## Example 1. Isolation of plasmid pTY1 from Propionibacterium pentosaceum ATCC 4875 (IFO 12425)

A test tube (18 mm$\phi$ x 180 mm length) containing 10 ml of medium as described in Table 1 was inoculated with cells of Propionibacterium pentosaceum ATCC 4875 (IFO 12425) (FERM BP-2931), and the cells were cultured at 30°C for 2 days by a stationary culture to prepare an innoculum culture.

Then, 400 ml of a medium having a composition as described in Table 1 was placed in a 1 $\ell$ Sakaguchi's flask, and autoclaved and cooled. The fresh medium was inoculated with the above-prepared inoculum culture, and the cells were cultured at 30°C by a stationary culture. When an optical density (OD) of the culture reached 1.0, as measured at 610 nm, 2 units/ml of penicillin G was added to the culture medium, and the culturing was continued at 30°C for a further 2.5 hours. The cultured broth was centrifuged to recover cells, which were then washed with a TES buffer comprising 0.03 M tris-(hydroxymethyl)aminomethane (Tris), 0.005 M EDTA, and 0.05 M NaCl (pH 8.0), suspended in 20 ml of a lysozyme solution (25% sucrose, 0.1 M NaCl, 0.05 M Tris, 5 mg/ml lysozyme, pH 8.0), and the suspension was incubated at 37°C for one hour. Then, 2.4 ml of 5 M NaCl, 0.6 ml of 0.5 M EDTA (pH 8.5), and 4.4 ml of a solution comprising 0.4% sodium lauryl sulfate and 0.7 M NaCl were added sequentially to the suspension in this order, and the mixture was gently stirred in ice water for 15 hours. The whole lysate was then transferred to a centrifugation tube, and centrifuged at 4°C and 69400Xg for 60 minutes to obtain a supernatant. Then, 10% by weight of polyethylene glycol 6000 was added to the supernatant, and the mixture was gently stirred in ice water.

After 16 hours, the mixture was centrifuged for 10 minutes at 1500Xg to recover a pellet, which was then re-dissolved in 5 ml of TES buffer. Thereafter, 0.5 ml of 5 mg/ml ethidium bromide was added to the solution, and solid cesium chloride was gradually added to the resulting mixture to adjust the density of the solution to 1.580. This solution was centrifuged at 105,000Xg and 20°C for 48 hours, and after this density-gradient centrifugation, a covalently closed circular DNA was detected by UV radiation as a higher density band positioned at a lower portion of the centrifugation tube. This band was removed through the side wall of the tube, using a syringe, to obtain a fraction containing the plasmid pTY1.

Next, the fraction was 5 times extracted with the same volume of isopropanol solution (90% by volume of isopropanol and 10% by volume of TES buffer) saturated with cesium bromide to eliminate ethidium bromide, and then dialyzed against TEB buffer.

Next, to 1 ml of the dialysate containing the plasmid pTY1 thus obtained was added 2 ml of ethanol, and the resulting precipitate was filtered off and dried in vacuum at -20°C, to obtain 50 $\mu$g of the plasmid pTY1.

The plasmid pTY1 was subjected to the cleavage with BamH I, EcoR I, Sph I, Xho I, Hind III, Bgl I, Pst I or Xba I alone, or a combination of two or three restriction enzymes. The restriction enzymes were commercially available from Takara Shuzo, Japan, or Toyobo, Japan, and the digestion conditions followed those given by the maker. The resulting digestion product was subjected to agarose gel electrophoresis, and the molecular weight of a DNA fragment obtained by comparing its mobility with that of molecular weight markers prepared by digesting λ phage DNA with Hind III (see, Method in Enzymology Vol 12, Part B, pp 361 to 377, 1968, Academic Press. New York, USA).

As a result of more than ten repetitions of these experiments, the molecular weight of the plasmid pTY1 was found to be about 4.9 mega Dalton. A restriction enzyme cleavage map was determined as shown in Fig. 1.

## Claims

1. Plasmid pTY1 having a molecular weight of about 4.9 mega Dalton and defined by a restriction enzyme cleavage map shown in Fig. 1.

2. A plasmid according to claim 1, and obtainable from cells of Propionibacterium pentosaceum ATCC 4875 (IFO 12425) (FERM BP-2931).

# Fig. 1

BamHI(0/4.9)
(4.44)EcoRI
(4.11)EcoRI
(3.71)PstI*
Xhol(0.46)
pTY1
SphI(1.65)

**European Patent Office**

# EUROPEAN SEARCH REPORT

Application Number

EP   90 30 5756

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | Abstracts of the Annual meeting of the American Society of Microbiology; Vol.86 (1986), page 27 7Abst. no P-11; REHBERGER, T.G. & GLATZ, B.A. : "Restriction endonuclease analysis of Propioni-bacterium plasmids." *The whole abstract* | 1-2 | C12N15/74 |
| P,X | Applied & Environ. Microbiol. vol. 56, no. 4, April 1990, American Society for Microbiology pages 864 - 871; REHBERGER, T.G. et al.: "Characterization of Propionibacterium plasmids" * page 866; figure 1 *and table 2* | 1-2 | |

TECHNICAL FIELDS SEARCHED (Int. Cl.5)

C12N

The present search report has been drawn up for all claims

| Place of search | Date of completion of the search | Examiner |
|---|---|---|
| THE HAGUE | 20. AUGUST 1990 | ANDRES S.M. |

EPO FORM 1503 03.82 (P0401)